# DEMANDE DE BREVET EUROPEEN

(11) **EP 4 714 432 A1**
(43) Date de publication de la demande: **25.03.2026**
(21) Numéro de dépôt: 25203780.9
(22) Date de dépôt: 22.09.2025
(51) Int. Cl.: A61K 9/08, A61K 47/02, A61K 33/00, A61K 9/00, A61K 45/06

(54) **COMPOSITION IONIQUE À BASE D'EAU DE MER POUR LA FABRICATION DE DISPOSITIFS MÉDICAUX ET DE COSMÉTIQUES À VISÉE RÉPARATRICE ET APAISANTE**

(30) Priorité: 20.09.2024 FR 2410056
(71) Demandeur: Laboratoires Chemineau, 37210 Vouvray (FR)
(72) Inventeur: BEAULIEU, Anne, 37210 Dinard (FR); LE TAILLANDIER DE GABORY, Ludovic, 33000 Bordeaux (FR)
(74) Mandataire: Yes My Patent

(57) **Abrégé**

L'invention concerne une composition ionique à base d'eau de mer pour la fabrication de dispositifs médicaux destinés à l'administration de ladite composition chez des patients, et de cosmétiques à visée réparatrice et apaisante, comprenant une concentration particulière en chlorure (Cl), sodium (Na), sulfate (SO4), soufre (S), magnésium (Mg), calcium (Ca), potassium (K), bicarbonate (HCO3). Cette composition est reproductible, stable, bien tolérée et peut être utilisée seule ou en combinaison avec d'autres ingrédients.

## Description

L'invention concerne une composition ionique à base d'eau de mer pour la fabrication de dispositifs médicaux destinés à l'administration de ladite composition chez des patients, et de cosmétiques à visée réparatrice et apaisante, comprenant une concentration particulière en chlorure (Cl), sodium (Na), sulfate (SO4), soufre (S), magnésium (Mg), calcium (Ca), potassium (K), bicarbonate (HCO3). Cette composition est reproductible, stable, bien tolérée et peut être utilisée seule ou en combinaison avec d'autres ingrédients.

### Art Antérieur

On constate dans un certain nombre de pathologies inflammatoires chroniques impliquant les muqueuses et/ou la peau un lien entre l'augmentation du stress oxydatif, l'altération de la fonction barrière à l'origine de microlésions et des dysfonctionnements des processus de l'immunité locale.

La perméabilité étant un facteur clef du déclenchement des mécanismes inflammatoires, on comprend mieux l'intérêt de limiter le stress oxydatif et de préserver ou favoriser la fonction barrière de la peau et des muqueuses.

En ORL, on sait que les principaux composants de la pollution atmosphérique peuvent induire un stress oxydatif et des réponses inflammatoires dans les cellules épithéliales nasales (Hong, 2016). Le contact prolongé avec les polluants et allergènes atmosphériques, ou avec les pathogènes respiratoires entraîne une inflammation pulmonaire et des lésions de l'épithélium respiratoire.

Les bénéfices de l'eau de mer sont connus et documentés sur l'ensemble des pathologies respiratoires hautes mais beaucoup moins sur les voies respiratoires basses, les autres muqueuses et la peau. *In vitro,* une expérimentation sur la muqueuse respiratoire (Bonnomet 2016) a montré, sur la fréquence des battements ciliaires et la vitesse de réparation des plaies de l'épithélium nasal, l'impact des électrolytes dans les pathologies respiratoires et en suivi post-chirurgical. Les eaux de mer utilisées étaient isotoniques, et obtenues par deux procédés de fabrication différents, l'un par électrodialyse, l'autre par dilution. Une différence en faveur du premier procédé était montrée sur 14 sujets. Cette même étude a montré l'impact délétère des solutions de chlorure de sodium.

Cependant, les mécanismes d'action de l'eau de mer et l'impact de ses principaux électrolytes, en particulier le magnésium, le calcium et le soufre, sont peu connus sur la muqueuse respiratoire, sur les muqueuses plus généralement et davantage encore sur la peau. La richesse minérale de l'eau de mer naturelle qui totalise plus de 80 ions différents dans une seule matrice en fait un ingrédient remarquable pour des utilisations non seulement dans la sphère respiratoire mais aussi dans toutes les sphères adjacentes, telle que l'appareil buccopharyngé et l'appareil pulmonaire, mais aussi en utilisation sur d'autres muqueuses et sur la peau.

La Demanderesse a de manière surprenante identifié des compositions à base d'eau de mer dont les résultats présentés en exemple démontrent l'inhibition de 7 marqueurs de l'inflammation, les interleukines IL8, IL6, IL18, IL5, les cytokines TSLP (Thymic Stromal LymphoPoietin), GM-CSF (Granulocyte-Macrophage Colony-Stimulating Factor), la chiomiokine CCL26 (C-C Motif Chemokine Ligand 26), appelée également éotaxine-3.

Avantageusement, la demanderesse a également identifié des compositions qui associent des propriétés apaisantes et anti-inflammatoires telles que décrites ci-dessus à des propriétés cicatricielles mises en évidence par la mesure de la vitesse de réparation des tissus en µm²/heure.

### Description de l'invention

Ainsi, selon un premier aspect, l'invention concerne une composition à base d'eau de mer comprenant:
- un pH de 7 à 8 ;
- une conductivité de 15 à 19,5 mS/cm ;
- une osmolarité de 280 à 370 mOsm/kg, de préférence de 290 à 360 mOsm/kg ; et comprenant la teneur suivante en les principaux constituants :
- 2100 à 3900 mg/L de sodium (Na)
- 6000 à 7000 mg/L de chlorure (Cl)
- 20 à 120 mg/L de bicarbonate (HCO₃)
- 400 à 1900 mg/L de magnésium (Mg) ;
- 100 à 650 mg/L de calcium (Ca) ;
- 45 à 140 mg/L de potassium (K) ;
- 700 à 2600 mg/L de sulfate (SO₄).

Selon un mode de réalisation, la composition comprend en outre :
- 200 à 3000 mg/L de soufre (S).

Selon un mode de réalisation, la composition selon l'invention comprend en outre au moins un parmi : du zinc (Zn), du cuivre (Cu), du sélénium (Se), du manganèse (Mn), du fer (Fe).

De manière préférée, le pH de la composition est compris entre 7,2 et 7,8.

Selon un mode de réalisation, le pH de la composition est de 7,5.

Selon un mode de réalisation, le pH de la composition est de 7,8.

Selon un mode de réalisation, le pH de la composition est de 8.

De manière préférée, la composition a une osmolarité de 310 à 360 mOsm/kg.

Selon un mode de réalisation, la composition a une osmolarité de 315 mOsm/kg.

Selon un mode de réalisation, la composition a une osmolarité de 351 mOsm/kg.

Selon un mode de réalisation, la composition a une osmolarité de 332 mOsm/kg.

De manière préférée, la composition a une conductivité de 17,5 à 19,5 mS/cm.

Selon un mode de réalisation, la composition a une conductivité de 17,7 mS/cm.

Selon un mode de réalisation, la composition a une conductivité de 18,7 mS/cm.

Selon un mode de réalisation, la composition a une conductivité de 19 mS/cm.

Selon un mode de réalisation, la composition comprend une teneur en chlorure (Cl) choisie parmi : 6000 mg/L ou 6010 mg/L ou 6020 mg/L ou 6030 mg/L ou 6040 mg/L ou 6050 mg/L ou 6060 mg/L ou 6070 mg/L ou 6080 mg/L ou 6090 mg/L ou 6100 mg/L ou 6110 mg/L ou 6120 mg/L ou 6130 mg/L ou 6140 mg/L ou 6150 mg/L ou 6160 mg/L ou 6170 mg/L ou 6180 mg/L ou 6190 mg/L ou 6200 mg/L ou 6210 mg/L ou 6220 mg/L ou 6230 mg/L ou 6240 mg/L ou 6250 mg/L ou 6260 mg/L ou 6270 mg/L ou 6280 mg/L ou 6290 mg/L ou 6300 mg/L ou 6310 mg/L ou 6320 mg/L ou 6330 mg/L ou 6340 mg/L ou 6350 mg/L ou 6360 mg/L ou 6370 mg/L ou 6380 mg/L ou 6390 mg/L ou 6400 mg/L ou 6410 mg/L ou 6420 mg/L ou 6430 mg/L ou 6440 mg/L ou 6450 mg/L ou 6460 mg/L ou 6470 mg/L ou 6480 mg/L ou 6490 mg/L ou 6500 mg/L ou 6510 mg/L ou 6520 mg/L ou 6530 mg/L ou 6540 mg/L ou 6550 mg/L ou 6560 mg/L ou 6570 mg/L ou 6580 mg/L ou 6590 mg/L ou 6600 mg/L ou 6540 mg/L ou 6550 mg/L ou 6560 mg/L ou 6570 mg/L ou 6580 mg/L ou 6590 mg/L ou 6600 mg/L ou 6610 mg/L ou 6620 mg/L ou 6630 mg/L ou 6640 mg/L ou 6650 mg/L ou 6660 mg/L ou 6670 mg/L ou 6680 mg/L ou 6690 mg/L ou 6700 mg/L ou 6710 mg/L ou 6720 mg/L ou 6730 mg/L ou 6740 mg/L ou 6750 mg/L ou 6760 mg/L ou 6770 mg/L ou 6780 mg/L ou 6790 mg/L ou 6800 mg/L ou 6810 mg/L ou 6820 mg/L ou 6830 mg/L ou 6840 mg/L ou 6850 mg/L ou 6860 mg/L ou 6870 mg/L ou 6880 mg/L ou 6890 mg/L ou 6900 mg/L ou 6910 mg/L ou 6920 mg/L ou 6930 mg/L ou 6940 mg/L ou 6950 mg/L ou 6960 mg/L ou 6970 mg/L ou 6980 mg/L ou 6990 mg/L ou 7000 mg/L.

De manière préférée, la composition comprend 6000 à 6600 mg/L de chlorure (Cl).

Selon un mode de réalisation, la composition comprend 6100 à 6400 mg/L de chlorure (Cl).

Selon un mode de réalisation, la composition comprend 6150 à 6250 mg/L de chlorure (Cl), de préférence 6200 mg/L.

Selon un mode de réalisation, la composition comprend 6300 à 6600 mg/L de chlorure (Cl).

Selon un mode de réalisation, la composition comprend 6400 à 6500 mg/L de chlorure (Cl), de préférence 6471 mg/L.

Selon un mode de réalisation, la composition comprend 6900 à 7000 mg/L de chlorure (Cl).

Selon un mode de réalisation, la composition comprend 6900 à 6950 mg/L de chlorure (Cl), de préférence 6917 mg/L.

Selon un mode de réalisation, la composition comprend une teneur en sodium (Na) choisie parmi : 2500 mg/L ou 2510 mg/L ou 2520 mg/L ou 2530 mg/L ou 2540 mg/L ou 2550 mg/L ou 2560 mg/L ou 2570 mg/L ou 2580 mg/L ou 2590 mg/L ou 2600 mg/L ou 2610 mg/L ou 2620 mg/L ou 2630 mg/L ou 2640 mg/L ou 2650 mg/L ou 2660 mg/L ou 2670 mg/L ou 2680 mg/L ou 2690 mg/L ou 2700 mg/L ou 2710 mg/L ou 2720 mg/L ou 2730 mg/L ou 2740 mg/L ou 2750 mg/L ou 2760 mg/L ou 2770 mg/L ou 2780 mg/L ou 2790 mg/L ou 2800 mg/L ou 2810 mg/L ou 2820 mg/L ou 2830 mg/L ou 2840 mg/L ou 2850 mg/L ou 2860 mg/L ou 2870 mg/L ou 2880 mg/L ou 2890 mg/L ou 2900 mg/L ou 2910 mg/L ou 2920 mg/L ou 2930 mg/L ou 2940 mg/L ou 2950 mg/L ou 2960 mg/L ou 2970 mg/L ou 2980 mg/L ou 2990 mg/L ou 3000 mg/L ou 3010 mg/L ou 3020 mg/L ou 3030 mg/L ou 3040 mg/L ou 3050 mg/L ou 3060 mg/L ou 3070 mg/L ou 3080 mg/L ou 3090 mg/L ou 3100 mg/L ou 3110 mg/L ou 3120 mg/L ou 3130 mg/L ou 3140 mg/L ou 3150 mg/L ou 3160 mg/L ou 3170 mg/L ou 3180 mg/L ou 3190 mg/L ou 3200 mg/L ou 3210 mg/L ou 3220 mg/L ou 3230 mg/L ou 3240 mg/L ou 3250 mg/L ou 3260 mg/L ou 3270 mg/L ou 3280 mg/L ou 3290 mg/L ou 3300 mg/L ou 3310 mg/L ou 3320 mg/L ou 3330 mg/L ou 3340 mg/L ou 3350 mg/L ou 3360 mg/L ou 3370 mg/L ou 3380 mg/L ou 3390 mg/L ou 3400 mg/L ou 3410 mg/L ou 3420 mg/L ou 3430 mg/L ou 3440 mg/L ou 3450 mg/L ou 3460 mg/L ou 3470 mg/L ou 3480 mg/L ou 3490 mg/L ou 3500 mg/L ou 3510 mg/L ou 3520 mg/L ou 3530 mg/L ou 3540 mg/L ou 3550 mg/L ou 3560 mg/L ou 3570 mg/L ou 3580 mg/L ou 3590 mg/L ou 3600 mg/L ou 3610 mg/L ou 3620 mg/L ou 3630 mg/L ou 3640 mg/L ou 3650 mg/L ou 3660 mg/L ou 3670 mg/L ou 3680 mg/L ou 3690 mg/L ou 3700 mg/L ou 3710 mg/L ou 3720 mg/L ou 3730 mg/L ou 3740 mg/L ou 3750 mg/L ou 3760 mg/L ou 3770 mg/L ou 3780 mg/L ou 3790 mg/L ou 3800 mg/L.

De manière préférée, la composition comprend 2500 à 3800 mg/L de sodium (Na).

Selon un mode de réalisation, la composition comprend 3550 à 3700 mg/L de sodium (Na), de préférence 3600 ou 3669 mg/L.

Selon un mode de réalisation, la composition comprend 2700 à 2900 mg/L de sodium (Na), de préférence 2786 mg/L.

Selon un mode de réalisation, la composition comprend une teneur en bicarbonate (HCO₃⁻) choisie parmi : 20 mg/L ou 21 mg/L ou 22 mg/L ou 23 mg/L ou 24 mg/L ou 25 mg/L ou 26 mg/L ou 27 mg/L ou 28 mg/L ou 29 mg/L ou 30 mg/L ou 31 mg/L ou 32 mg/L ou 33 mg/L ou 34 mg/L ou 35 mg/L ou 36 mg/L ou 37 mg/L ou 38 mg/L ou 39 mg/L ou 40 mg/L ou 41 mg/L ou 42 mg/L ou 43 mg/L ou 44 mg/L ou 45 mg/L ou 46 mg/L ou 47 mg/L ou 48 mg/L ou 49 mg/L ou 50 mg/L ou 51 mg/L ou 52 mg/L ou 53 mg/L ou 54 mg/L ou 55 mg/L ou 56 mg/L ou 57 mg/L ou 58 mg/L ou 59 mg/L ou 60 mg/L ou 61 mg/L ou 62 mg/L ou 63 mg/L ou 64 mg/L ou 65 mg/L ou 66 mg/L ou 67 mg/L ou 68 mg/L ou 69 mg/L ou 70 mg/L ou 71 mg/L ou 72 mg/L ou 73 mg/L ou 74 mg/L ou 75 mg/L ou 76 mg/L ou 77 mg/L ou 78 mg/L ou 79 mg/L ou 80 mg/L ou 81 mg/L ou 82 mg/L ou 83 mg/L ou 84 mg/L ou 85 mg/L ou 86 mg/L ou 87 mg/L ou 88 mg/L ou 89 mg/L ou 90 mg/L ou 91 mg/L ou 92 mg/L ou 93 mg/L ou 94 mg/L ou 95 mg/L ou 96 mg/L ou 97 mg/L ou 98 mg/L ou 99 mg/L ou 100 mg/L ou 101 mg/L ou 102 mg/L ou 103 mg/L ou 104 mg/L ou 105 mg/L ou 106 mg/L ou 107 mg/L ou 108 mg/L ou 109 mg/L ou 110 mg/L ou 111 mg/L ou 112 mg/L ou 113 mg/L ou 114 mg/L ou 115 mg/L ou 116 mg/L ou 117 mg/L ou 118 mg/L ou 119 mg/L ou 120 mg/L.

Selon un mode de réalisation, la composition comprend 35 à 45 mg/L de bicarbonate (HCO3), de préférence 39 mg/L.

Selon un mode de réalisation, la composition comprend 100 à 120 mg/L de bicarbonate (HCO3), de préférence 119 mg/L.

Selon un mode de réalisation, la composition comprend une teneur en magnésium (Mg) choisie parmi : 400 mg/L ou 410 mg/L ou 420 mg/L ou 430 mg/L ou 440 mg/L ou 450 mg/L ou 460 mg/L ou 470 mg/L ou 480 mg/L ou 490 mg/L ou 500 mg/L ou 510 mg/L ou 520 mg/L ou 530 mg/L ou 540 mg/L ou 550 mg/L ou 560 mg/L ou 570 mg/L ou 580 mg/L ou 590 mg/L ou 600 mg/L ou 610 mg/L ou 620 mg/L ou 630 mg/L ou 640 mg/L ou 650 mg/L ou 660 mg/L ou 670 mg/L ou 680 mg/L ou 690 mg/L ou 700 mg/L ou 710 mg/L ou 720 mg/L ou 730 mg/L ou 740 mg/L ou 750 mg/L ou 760 mg/L ou 770 mg/L ou 780 mg/L ou 790 mg/L ou 800 mg/L ou 810 mg/L ou 820 mg/L ou 830 mg/L ou 840 mg/L ou 850 mg/L ou 860 mg/L ou 870 mg/L ou 880 mg/L ou 890 mg/L ou 900 mg/L ou 910 mg/L ou 920 mg/L ou 930 mg/L ou 940 mg/L ou 950 mg/L ou 960 mg/L ou 970 mg/L ou 980 mg/L ou 990 mg/L ou 1000 mg/L ou 1010 mg/L ou 1020 mg/L ou 1030 mg/L ou 1040 mg/L ou 1050 mg/L ou 1060 mg/L ou 1070 mg/L ou 1080 mg/L ou 1090 mg/L ou 1100 mg/L ou 1110 mg/L ou 1120 mg/L ou 1130 mg/L ou 1140 mg/L ou 1150 mg/L ou 1160 mg/L ou 1170 mg/L ou 1180 mg/L ou 1190 mg/L ou 1200 mg/L ou 1210 mg/L ou 1220 mg/L ou 1230 mg/L ou 1240 mg/L ou 1250 mg/L ou 1260 mg/L ou 1270 mg/L ou 1280 mg/L ou 1290 mg/L ou 1300 mg/L ou 1310 mg/L ou 1320 mg/L ou 1330 mg/L ou 1340 mg/L ou 1350 mg/L ou 1360 mg/L ou 1370 mg/L ou 1380 mg/L ou 1390 mg/L ou 1400 mg/L ou 1410 mg/L ou 1420 mg/L ou 1430 mg/L ou 1440 mg/L ou 1450 mg/L ou 1460 mg/L ou 1470 mg/L ou 1480 mg/L ou 1490 mg/L ou 1500 mg/L ou 1510 mg/L ou 1520 mg/L ou 1530 mg/L ou 1540 mg/L ou 1550 mg/L ou 1560 mg/L ou 1570 mg/L ou 1580 mg/L ou 1590 mg/L ou 1600 mg/L ou 1610 mg/L ou 1620 mg/L ou 1630 mg/L ou 1640 mg/L ou 1650 mg/L ou 1660 mg/L ou 1670 mg/L ou 1680 mg/L ou 1690 mg/L ou 1700 mg/L ou 1710 mg/L ou 1720 mg/L ou 1730 mg/L ou 1740 mg/L ou 1750 mg/L ou 1760 mg/L ou 1770 mg/L ou 1780 mg/L ou 1790 mg/L ou 1800 mg/L ou 1810 mg/L ou 1820 mg/L ou 1830 mg/L ou 1840 mg/L ou 1850 mg/L ou 1860 mg/L ou 1870 mg/L ou 1880 mg/L ou 1890 mg/L ou 1900 mg/L.

Selon un mode de réalisation, la composition comprend 350 à 1000 mg/L de magnésium (Mg).

Selon un mode de réalisation, la composition comprend 350 à 500 mg/L de magnésium (Mg), de préférence 400 et à 450 mg/L, de manière encore plus préférée 426 mg/L.

Selon un mode de réalisation, la composition comprend 800 à 1000 mg/L de magnésium (Mg), de préférence 900 et à 950 mg/L, de manière encore plus préférée 923 mg/L.

Selon un autre mode de réalisation, la composition comprend 1400 à 1600 mg/L de magnésium (Mg), de manière préférée 1509 mg/L.

Selon un mode de réalisation, la composition comprend une teneur en calcium (Ca) choisie parmi :100 mg/L ou 110 mg/L ou 120 mg/L ou 130 mg/L ou 140 mg/L ou 150 mg/L ou 160 mg/L ou 170 mg/L ou 180 mg/L ou 190 mg/L ou 200 mg/L ou 210 mg/L ou 220 mg/L ou 230 mg/L ou 240 mg/L ou 250 mg/L ou 260 mg/L ou 270 mg/L ou 280 mg/L ou 290 mg/L ou 300 mg/L ou 310 mg/L ou 320 mg/L ou 330 mg/L ou 340 mg/L ou 350 mg/L ou 360 mg/L ou 370 mg/L ou 380 mg/L ou 390 mg/L ou 400 mg/L ou 410 mg/L ou 420 mg/L ou 430 mg/L ou 440 mg/L ou 450 mg/L ou 460 mg/L ou 470 mg/L ou 480 mg/L ou 490 mg/L ou 500 mg/L ou 510 mg/L ou 520 mg/L ou 530 mg/L ou 540 mg/L ou 550 mg/L ou 560 mg/L ou 570 mg/L ou 580 mg/L ou 590 mg/L ou 600 mg/L ou 610 mg/L ou 620 mg/L ou 630 mg/L ou 640 mg/L ou 650 mg/L.

De manière préférée, la composition comprend 100 à 400 mg/L de calcium (Ca).

Selon un mode de réalisation, la composition comprend 100 à 150 mg/L de calcium (Ca), de préférence 123 mg/L.

Selon un mode de réalisation, la composition comprend 250 à 325 mg/L de calcium (Ca), de préférence 290 mg/L.

Selon un mode de réalisation, la composition comprend 370 à 400 mg/L de calcium (Ca), de préférence 390 mg/L.

Selon un mode de réalisation, la composition comprend une teneur en potassium (K) choisie parmi : 45 mg/L ou 46 mg/L ou 47 mg/L ou 48 mg/L ou 49 mg/L ou 50 mg/L ou 51 mg/L ou 52 mg/L ou 53 mg/L ou 54 mg/L ou 55 mg/L ou 56 mg/L ou 57 mg/L ou 58 mg/L ou 59 mg/L ou 60 mg/L ou 61 mg/L ou 62 mg/L ou 63 mg/L ou 64 mg/L ou 65 mg/L ou 66 mg/L ou 67 mg/L ou 68 mg/L ou 69 mg/L ou 70 mg/L ou 71 mg/L ou 72 mg/L ou 73 mg/L ou 74 mg/L ou 75 mg/L ou 76 mg/L ou 77 mg/L ou 78 mg/L ou 79 mg/L ou 80 mg/L ou 81 mg/L ou 82 mg/L ou 83 mg/L ou 84 mg/L ou 85 mg/L ou 86 mg/L ou 87 mg/L ou 88 mg/L ou 89 mg/L ou 90 mg/L ou 91 mg/L ou 92 mg/L ou 93 mg/L ou 94 mg/L ou 95 mg/L ou 96 mg/L ou 97 mg/L ou 98 mg/L ou 99 mg/L ou 100 mg/L ou 101 mg/L ou 102 mg/L ou 103 mg/L ou 104 mg/L ou 105 mg/L ou 106 mg/L ou 107 mg/L ou 108 mg/L ou 109 mg/L ou 110 mg/L ou 111 mg/L ou 112 mg/L ou 113 mg/L ou 114 mg/L ou 115 mg/L ou 116 mg/L ou 117 mg/L ou 118 mg/L ou 119 mg/L ou 120 mg/L ou 121 mg/L ou 122 mg/L ou 123 mg/L ou 124 mg/L ou 125 mg/L ou 126 mg/L ou 127 mg/L ou 128 mg/L ou 129 mg/L ou 130 mg/L ou 131 mg/L ou 132 mg/L ou 133 mg/L ou 134 mg/L ou 135 mg/L ou 136 mg/L ou 137 mg/L ou 138 mg/L ou 139 mg/L ou 140 mg/L.

Selon un mode de réalisation, la composition comprend 100 à 150 mg/L de potassium (K).

Selon un mode de réalisation, la composition comprend 115 à 145 mg/L de potassium (K). De préférence, la composition comprend 122 ou 135 mg/L de potassium (K).

Selon un mode de réalisation, la composition comprend 60 à 80 mg/L de potassium (K), de préférence 71 mg/L.

Selon un mode de réalisation, la composition comprend une teneur en sulfate (SO₄) choisie parmi :700 mg/L ou 710 mg/L ou 720 mg/L ou 730 mg/L ou 740 mg/L ou 750 mg/L ou 760 mg/L ou 770 mg/L ou 780 mg/L ou 790 mg/L ou 800 mg/L ou 810 mg/L ou 820 mg/L ou 830 mg/L ou 840 mg/L ou 850 mg/L ou 860 mg/L ou 870 mg/L ou 880 mg/L ou 890 mg/L ou 900 mg/L ou 910 mg/L ou 920 mg/L ou 930 mg/L ou 940 mg/L ou 950 mg/L ou 960 mg/L ou 970 mg/L ou 980 mg/L ou 990 mg/L ou 1000 mg/L ou 1010 mg/L ou 1020 mg/L ou 1030 mg/L ou 1040 mg/L ou 1050 mg/L ou 1060 mg/L ou 1070 mg/L ou 1080 mg/L ou 1090 mg/L ou 1100 mg/L ou 1110 mg/L ou 1120 mg/L ou 1130 mg/L ou 1140 mg/L ou 1150 mg/L ou 1160 mg/L ou 1170 mg/L ou 1180 mg/L ou 1190 mg/L ou 1200 mg/L ou 1210 mg/L ou 1220 mg/L ou 1230 mg/L ou 1240 mg/L ou 1250 mg/L ou 1260 mg/L ou 1270 mg/L ou 1280 mg/L ou 1290 mg/L ou 1300 mg/L ou 1310 mg/L ou 1320 mg/L ou 1330 mg/L ou 1340 mg/L ou 1350 mg/L ou 1360 mg/L ou 1370 mg/L ou 1380 mg/L ou 1390 mg/L ou 1400 mg/L ou 1410 mg/L ou 1420 mg/L ou 1430 mg/L ou 1440 mg/L ou 1450 mg/L ou 1460 mg/L ou 1470 mg/L ou 1480 mg/L ou 1490 mg/L ou 1500 mg/L ou 1510 mg/L ou 1520 mg/L ou 1530 mg/L ou 1540 mg/L ou 1550 mg/L ou 1560 mg/L ou 1570 mg/L ou 1580 mg/L ou 1590 mg/L ou 1600 mg/L ou 1610 mg/L ou 1620 mg/L ou 1630 mg/L ou 1640 mg/L ou 1650 mg/L ou 1660 mg/L ou 1670 mg/L ou 1680 mg/L ou 1690 mg/L ou 1700 mg/L ou 1710 mg/L ou 1720 mg/L ou 1730 mg/L ou 1740 mg/L ou 1750 mg/L ou 1760 mg/L ou 1770 mg/L ou 1780 mg/L ou 1790 mg/L ou 1800 mg/L ou 1810 mg/L ou 1820 mg/L ou 1830 mg/L ou 1840 mg/L ou 1850 mg/L ou 1860 mg/L ou 1870 mg/L ou 1880 mg/L ou 1890 mg/L ou 1900 mg/L ou 1910 mg/L ou 1920 mg/L ou 1930 mg/L ou 1940 mg/L ou 1950 mg/L ou 1960 mg/L ou 1970 mg/L ou 1980 mg/L ou 1990 mg/L ou 2000 mg/L ou 2010 mg/L ou 2020 mg/L ou 2030 mg/L ou 2040 mg/L ou 2050 mg/L ou 2060 mg/L ou 2070 mg/L ou 2080 mg/L ou 2090 mg/L ou 2100 mg/L ou 2110 mg/L ou 2120 mg/L ou 2130 mg/L ou 2140 mg/L ou 2150 mg/L ou 2160 mg/L ou 2170 mg/L ou 2180 mg/L ou 2190 mg/L ou 2200 mg/L ou 2210 mg/L ou 2220 mg/L ou 2230 mg/L ou 2240 mg/L ou 2250 mg/L ou 2260 mg/L ou 2270 mg/L ou 2280 mg/L ou 2290 mg/L ou 2300 mg/L ou 2310 mg/L ou 2320 mg/L ou 2330 mg/L ou 2340 mg/L ou 2350 mg/L ou 2360 mg/L ou 2370 mg/L ou 2380 mg/L ou 2390 mg/L ou 2400 mg/L ou 2410 mg/L ou 2420 mg/L ou 2430 mg/L ou 2440 mg/L ou 2450 mg/L ou 2460 mg/L ou 2470 mg/L ou 2480 mg/L ou 2490 mg/L ou 2500 mg/L ou 2510 mg/L ou 2520 mg/L ou 2530 mg/L ou 2540 mg/L ou 2550 mg/L ou 2560 mg/L ou 2570 mg/L ou 2580 mg/L ou 2590 mg/L ou 2600 mg/L.

Selon un mode de réalisation, la composition comprend 750 à 1000 mg/L de sulfate (SO4), de préférence 800 à 950 mg/L de sulfate (SO4), de manière encore plus préférée 890 mg/L de sulfate (SO₄).

Selon un mode de réalisation, la composition comprend 1250 à 1500 mg/L de sulfate (SO4), de préférence 1300 à 1400 mg/L de sulfate (SO4), de manière encore plus préférée 1357 mg/L de sulfate (SO4).

Selon un mode de réalisation, la composition comprend 1800 à 2100 mg/L de sulfate (SO4), de préférence 1900 à 2000 mg/L de sulfate (SO4), de manière encore plus préférée 1973 mg/L de sulfate (SO4).

De manière préférée, la composition comprend entre 200 et 1000 mg/L de soufre (S).

Selon un mode de réalisation, la composition comprend une teneur en soufre (S) choisie parmi : 200 mg/L ou 210 mg/L ou 220 mg/L ou 230 mg/L ou 240 mg/L ou 250 mg/L ou 260 mg/L ou 270 mg/L ou 280 mg/L ou 290 mg/L ou 300 mg/L ou 310 mg/L ou 320 mg/L ou 330 mg/L ou 340 mg/L ou 350 mg/L ou 360 mg/L ou 370 mg/L ou 380 mg/L ou 390 mg/L ou 400 mg/L ou 410 mg/L ou 420 mg/L ou 430 mg/L ou 440 mg/L ou 450 mg/L ou 460 mg/L ou 470 mg/L ou 480 mg/L ou 490 mg/L ou 500 mg/L ou 510 mg/L ou 520 mg/L ou 530 mg/L ou 540 mg/L ou 550 mg/L ou 560 mg/L ou 570 mg/L ou 580 mg/L ou 590 mg/L ou 600 mg/L ou 610 mg/L ou 620 mg/L ou 630 mg/L ou 640 mg/L ou 650 mg/L ou 660 mg/L ou 670 mg/L ou 680 mg/L ou 690 mg/L ou 700 mg/L ou 710 mg/L ou 720 mg/L ou 730 mg/L ou 740 mg/L ou 750 mg/L ou 760 mg/L ou 770 mg/L ou 780 mg/L ou 790 mg/L ou 800 mg/L ou 810 mg/L ou 820 mg/L ou 830 mg/L ou 840 mg/L ou 850 mg/L ou 860 mg/L ou 870 mg/L ou 880 mg/L ou 890 mg/L ou 900 mg/L ou 910 mg/L ou 920 mg/L ou 930 mg/L ou 940 mg/L ou 950 mg/L ou 960 mg/L ou 970 mg/L ou 980 mg/L ou 990 mg/L ou 1000 mg/L.

Selon un mode de réalisation, la composition comprend entre 200 et 400 mg/L de soufre (S), de préférence 250 à 350 mg/L de soufre (S), de manière encore plus préférée 285 mg/L de soufre (S).

Selon un mode de réalisation, la composition comprend entre 500 et 700 mg/L de soufre (S), de préférence 550 à 650 mg/L de soufre (S), de manière encore plus préférée 600 mg/L de soufre (S).

Selon un mode de réalisation, la composition comprend entre 700 et 900 mg/L de soufre (S), de préférence 750 à 800 mg/L de soufre (S), de manière encore plus préférée 764 mg/L de soufre (S).

Selon un mode de réalisation, la composition a pour principaux constituants :
- 3600 mg/L de sodium (Na) ;
- 6200 mg/L de chlorure (Cl) ;
- 39 mg/L de bicarbonate (HCO₃)
- 426 mg/L de magnésium (Mg) ;
- 123 mg/L de calcium (Ca) ;
- 122 mg/L de potassium (K) ;
- 890 mg/L de sulfate (SO₄) ;
- 285 mg/L de soufre (S).

Cette composition a un pH de 7,5, une conductivité de 17,7 mS/cm, et une osmolarité de 315 mOsm/kg.

Selon un mode de réalisation, la composition a pour principaux constituants :
- 3669 mg/L de sodium (Na) ;
- 6471 mg/L de chlorure (Cl) ;
- 39 mg/L de bicarbonate (HCO₃)
- 923 mg/L de magnésium (Mg) ;
- 290 mg/L de calcium (Ca) ;
- 135 mg/L de potassium (K) ;
- 1357 mg/L de sulfate (SO₄) ;
- 600 mg/L de soufre (S).

Cette composition a un pH de 7,8, une conductivité de 18,7 mS/cm, et une osmolarité de 351 mOsm/kg.

Selon un mode de réalisation, la composition a pour principaux constituants :
- 2786 mg/L de sodium (Na) ;
- 6917 mg/L de chlorure (Cl) ;
- 119 mg/L de bicarbonate (HCO₃)
- 1509 mg/L de magnésium (Mg) ;
- 390 mg/L de calcium (Ca) ;
- 71 mg/L de potassium (K) ;
- 1973 mg/L de sulfate (SO₄) ;
- 764 mg/L de soufre (S).

Cette composition a un pH de 8, une conductivité de 19 mS/cm, et une osmolarité de 332 mOsm/kg.

Selon un mode de réalisation, la composition peut être formulée en spray nasal, de préférence sous forme de gouttelettes supérieures ou égales à 10 µm, de préférence pour une utilisation intranasale, buccale ou pharyngée.

Selon un mode de réalisation, la composition peut être formulée en nébulisation, de préférence sous forme de gouttelettes inférieures à 10µm, permettant de pénétrer dans l'arbre pulmonaire

Selon un mode de réalisation, la composition peut être formulée en suspension, gel, crème, émulsion et/ou pommade, de préférence avec une viscosité 100 à 1000 000 mPa·s à 25 °C.

La composition peut comprendre d'autres ingrédients d'intérêt notamment des actifs, solvants, corps gras, protecteurs, conservateurs, agents sensoriels, de libération de pénétration, et/ou des gaz propulseurs inertes (air, azote), et/ou gaz liquéfiés. Selon un autre aspect, l'invention concerne l'utilisation de la composition selon l'invention pour la fabrication d'un dispositif médical destiné à l'administration de ladite composition chez des patients.

Selon un mode de réalisation, ledit dispositif médical est utilisé dans le traitement ou la prévention des affections respiratoires en cas d'état inflammatoire, allergique ou dans la cicatrisation.

De manière préférée, ledit dispositif médical est utilisé dans le traitement ou la prévention de la polypose nasale, de la rhinite allergique, de l'asthme.

De manière préférée, ledit dispositif médical est utilisé dans le traitement ou la prévention des pathologies dermatologiques nécessitant un effet anti-inflammatoire et/ou de cicatrisation, par exemple la dermatite atopique.

Selon un mode de réalisation, ledit dispositif médical est utilisé dans le traitement ou la prévention de toutes affections bucco-pharyngées nécessitant un effet anti-inflammatoire et/ou de cicatrisation.

Selon un mode de réalisation, le dispositif médical est un nébuliseur (à jet, ultrasonique ou à membrane vibrante) ou est fourni en kit avec un tel nébuliseur, configuré pour générer un aérosol de diamètre aérodynamique massique médian (MMAD) >10 µm pour une administration aux voies respiratoires supérieures et/ou <10 µm pour une administration aux voies respiratoires inférieures.

Selon un mode de réalisation, le dispositif médical est destiné à une administration topique et comprend la composition formulée sous forme de, suspension, gel, crème, émulsion et/ou pommade, ladite formulation présentant de préférence une viscosité comprise entre 100 et 1 000 000 mPa.s à 25 °C pour assurer l'adhérence tissulaire et la stabilité d'usage.

Selon un mode de réalisation, le dispositif médical comprend d'autres ingrédients d'intérêt notamment des actifs, solvants, corps gras, protecteurs, conservateurs, agents sensoriels, de libération de pénétration, et/ou des gaz propulseurs inertes (air, azote), et/ou gaz liquéfiés

Selon un autre aspect, l'invention concerne un dispositif médical destiné à l'administration d'une composition ionique à base d'eau de mer selon l'invention.

Selon un mode de réalisation, ledit dispositif médical est utilisé pour le traitement ou la prévention des affections respiratoires en cas d'état inflammatoire, allergique ou dans la cicatrisation. Le dispositif médical est configuré pour une administration locale ou par inhalation.

Selon un mode de réalisation, le dispositif médical est destiné au traitement ou à la prévention de la polypose nasale, de la rhinite allergique ou de l'asthme. Le dispositif est conçu pour délivrer la composition de manière efficace sur les muqueuses respiratoires.

Selon un mode de réalisation, le dispositif médical est destiné au traitement ou à la prévention des pathologies dermatologiques nécessitant un effet anti-inflammatoire et/ou cicatrisant, notamment la dermatite atopique.

Selon un mode de réalisation, le dispositif médical est destiné au traitement ou à la prévention des affections bucco-pharyngées nécessitant un effet anti-inflammatoire et/ou cicatrisant.

Selon un mode de réalisation, le dispositif médical est choisi parmi : Spray nasal, système bag-on-valve, solution buvable, conditionnement unidose stérile, nébuliseur à jet, nébuliseur ultrasonique, nébuliseur à membrane vibrante, inhalateur doseur pressurisé, inhalateur de poudre sèche, tube ou flacon pour gel, crème, émulsion ou pommade, lingettes imprégnées, patch dermo-adhésif, film occlusif, compresse ou pansement imprégné, spray buccal, bain de bouche, solution de gargarisme, film orodispersible, comprimé muco-adhésif, forme solide (comprimé, poudre, etc..) kit combiné avec embout nasal ou nébuliseur, dispositif implantable ou insert muqueux.

Selon un autre aspect, l'invention concerne une composition pour utilisation selon l'invention, où ladite composition reconstituée est ensuite diluée dans de l'eau, de préférence purifiée, à un ratio de 3 % à 50 %, préférablement de 5% à 40% (m/m)

Selon un mode de réalisation, la composition pour utilisation selon l'invention est diluée dans de l'eau, de préférence purifiée (norme pharmacopée européenne), à un ratio (m/m) choisi parmi : 3.0 %, ou 3.5 %, ou 4.0 %, ou 4.5 %, ou 5.0 %, ou 5.5 %, ou 6.0 %, ou 6.5 %, ou 7.0 %, ou 7.5 %, ou 8.0 %, ou 8.5 %, ou 9.0 %, ou 9.5 %, ou 10.0 %, ou 10.5 %, ou 11.0 %, ou 11.5 %, ou 12.0 %, ou 12.5 %, ou 13.0 %, ou 13.5 %, ou 14.0 %, ou 14.5 %, ou 15.0 %, ou 15.5 %, ou 16.0 %, ou 16.5 %, ou 17.0 %, ou 17.5 %, ou 18.0 %, ou 18.5 %, ou 19.0 %, ou 19.5 %, ou 20.0 %, ou 20.5 %, ou 21.0 %, ou 21.5 %, ou 22.0 %, ou 22.5 %, ou 23.0 %, ou 23.5 %, ou 24.0 %, ou 24.5 %, ou 25.0 %, ou 25.5 %, ou 26.0 %, ou 26.5 %, ou 27.0 %, ou 27.5 %, ou 28.0 %, ou 28.5 %, ou 29.0 %, ou 29.5 %, ou 30.0 %, ou 30.5 %, ou 31.0 %, ou 31.5 %, ou 32.0 %, ou 32.5 %, ou 33.0 %, ou 33.5 %, ou 34.0 %, ou 34.5 %, ou 35.0 %, ou 35.5 %, ou 36.0 %, ou 36.5 %, ou 37.0 %, ou 37.5 %, ou 38.0 %, ou 38.5 %, ou 39.0 %, ou 39.5 %, ou 40.0 %, ou 40.5 %, ou 41.0 %, ou 41.5 %, ou 42.0 %, ou 42.5 %, ou 43.0 %, ou 43.5 %, ou 44.0 %, ou 44.5 %, ou 45.0 %, ou 45.5 %, ou 46.0 %, ou 46.5 %, ou 47.0 %, ou 47.5 %, ou 48.0 %, ou 48.5 %, ou 49.0 %, ou 49.5 %, ou 50.0 %.

Selon un mode de réalisation, ladite composition est pour utilisation pour favoriser la cicatrisation de tissus épithéliaux, de préférence de la muqueuse nasale.

Selon un autre aspect, l'invention concerne l'utilisation cosmétique de ladite composition chez un sujet sain.

Selon un mode de réalisation, ladite utilisation cosmétique est une utilisation non-thérapeutique.

Selon un mode de réalisation, ladite utilisation cosmétique est réalisée par administration par voie topique, sur la peau ou les muqueuses.

Selon un mode de réalisation, ladite utilisation cosmétique est réalisée par administration par voie topique, en application locale, de préférence par installation, spray continu, spray dosé, pulvérisation, brumisation, nébulisation, gargarisme, lavage, inspiration, suspension, gel, crème, émulsion, et/ou pommade.

Selon un mode de réalisation, l'utilisation cosmétique chez le sujet sain vise à rincer, nettoyer et/ou assainir la peau et/ou les muqueuses, à favoriser la réparation de la peau ou des muqueuses, à favoriser la fonction barrière de la peau ou des muqueuses, à favoriser la réduction du stress oxydatif de la peau et/ou des muqueuses, à soulager les rougeurs, gonflements, irritations, démangeaisons, suintements, grattages, ou sensations de brûlures.

Selon un autre aspect, l'invention concerne l'utilisation cosmétique non-thérapeutique chez un sujet sain de la composition selon l'invention, où ladite composition est diluée dans de l'eau, de préférence purifiée, à un ratio entre 3% et 50%, de préférence 5 % à 40(m/m).

Selon un mode de réalisation, ladite composition cosmétique non-thérapeutique est diluée dans de l'eau, de préférence purifiée (norme pharmacopée européenne), à un ratio (m/m) choisi parmi : 3.0 %, ou 3.5 %, ou 4.0 %, ou 4.5 %, ou 5.0 %, ou 5.5 %, ou 6.0 %, ou 6.5 %, ou 7.0 %, ou 7.5 %, ou 8.0 %, ou 8.5 %, ou 9.0 %, ou 9.5 %, ou 10.0 %, ou 10.5 %, ou 11.0 %, ou 11.5 %, ou 12.0 %, ou 12.5 %, ou 13.0 %, ou 13.5 %, ou 14.0 %, ou 14.5 %, ou 15.0 %, ou 15.5 %, ou 16.0 %, ou 16.5 %, ou 17.0 %, ou 17.5 %, ou 18.0 %, ou 18.5 %, ou 19.0 %, ou 19.5 %, ou 20.0 %, ou 20.5 %, ou 21.0 %, ou 21.5 %, ou 22.0 %, ou 22.5 %, ou 23.0 %, ou 23.5 %, ou 24.0 %, ou 24.5 %, ou 25.0 %, ou 25.5 %, ou 26.0 %, ou 26.5 %, ou 27.0 %, ou 27.5 %, ou 28.0 %, ou 28.5 %, ou 29.0 %, ou 29.5 %, ou 30.0 %, ou 30.5 %, ou 31.0 %, ou 31.5 %, ou 32.0 %, ou 32.5 %, ou 33.0 %, ou 33.5 %, ou 34.0 %, ou 34.5 %, ou 35.0 %, ou 35.5 %, ou 36.0 %, ou 36.5 %, ou 37.0 %, ou 37.5 %, ou 38.0 %, ou 38.5 %, ou 39.0 %, ou 39.5 %, ou 40.0 %, ou 40.5 %, ou 41.0 %, ou 41.5 %, ou 42.0 %, ou 42.5 %, ou 43.0 %, ou 43.5 %, ou 44.0 %, ou 44.5 %, ou 45.0 %, ou 45.5 %, ou 46.0 %, ou 46.5 %, ou 47.0 %, ou 47.5 %, ou 48.0 %, ou 48.5 %, ou 49.0 %, ou 49.5 %, ou 50.0 %.

Les compositions selon l'invention sont préférentiellement dépourvues ou comprennent de très faible quantité de tout agent conservateur ou stabilisant. Ce dernier avantage est d'une grande importance. En effet, les agents conservateurs et/ou stabilisants présents dans la plupart des compositions ioniques de synthèse, provoquent à plus ou moins long terme des effets secondaires. Or, selon l'invention, la composition est administrée sur des périodes de temps pouvant aller d'une semaine à plusieurs mois, voire à longueur d'années.

La composition selon l'invention a un effet apaisant et cicatrisant, comme caractérisé dans la section exemple. Cette composition a en outre un effet beaucoup plus rapide que les traitements habituels tels que les corticoïdes locaux.

Les résultats présentés en exemple démontrent l'inhibition de 7 marqueurs de l'inflammation, les interleukines IL8, IL6, IL18, IL5, les cytokines TSLP (Thymic Stromal LymphoPoietin), GM-CSF (Granulocyte-Macrophage Colony-Stimulating Factor), la chiomiokine CCL26 (C-C Motif Chemokine Ligand 26), appelée également éotaxine-3. Ces résultats sont présentés vs. contrôle en dexaméthasone (corticoïde puissant couramment utilisé pour ses propriétés anti-inflammatoires et immunosuppressives). La particularité des résultats est la diminution massive voire quasi-totale des sécrétions de ces facteurs d'inflammation obtenues avec ces solutions d'eau de mer vs. un médicament de référence, la dexaméthasone, le caractère global non spécifique des effets anti-inflammatoires observés et leur rapidité (moins de 24h). Ces compositions peuvent être utilisées pour réduire les marqueurs de l'inflammation IL8, IL6, TSLP, GM-CSF, CCL26, IL18, IL5 ou les marqueurs les plus souvent associés aux réactions allergiques ou à l'asthme ou la dermatite atopique comme le TSLP, CCL26, IL-5. Elles sont avantageuses pour rincer et assainir la peau et les muqueuses, favoriser la cicatrisation de la peau ou des muqueuses, favoriser la fonction barrière de la peau ou des muqueuses, favoriser la réduction du stress oxydatif de la peau ou des muqueuses, et soulager les signes et symptômes associées à des états inflammatoires ou allergiques tels que rougeur, gonflement, irritations, démangeaisons, suintements, grattage, ou sensations de brûlures.

Par rapport aux solutions à base d'eau de mer vendues aujourd'hui, les compositions objet de l'invention se différencient par l'abaissement de la teneur en chlore dans la solution d'eau de mer, et l'augmentation ou l'abaissement de minéraux d'intérêts, en particulier :
- le soufre et la famille des tampons (tel que les sulfates) qui font parties des ingrédients majeurs composant l'eau de mer ;
- mais également du calcium et du magnésium sous forme de sulfate de calcium, de lactate de magnésium ou de sulfate de magnésium ;
- Et de certains des oligo-éléments notamment zinc, cuivre, sélénium, fer, manganèse.

La particularité des solutions testées est d'avoir résolues différents problèmes de solubilisation, l'eau de mer tolérant très mal l'ajout d'ingrédient sans se troubler et créer toutes sortes de précipités. Elles peuvent notamment être utilisées seules, ou avec d'autres ingrédients miscibles.

Selon un autre aspect, l'invention concerne un kit comprenant:
- (i) une composition selon l'invention,;
- (ii) un dispositif d'administration, de préférence choisi parmi un système bag-on-valve (BOV), un conditionnement unidose ou un nébuliseur ;
- (iii) optionnellement, une notice précisant au moins une posologie topique adaptée à une pathologie ciblée.

Selon un mode de réalisation, le système BOV comprend un récipient métallique (par exemple en aluminium) renfermant un sac barrière souple soudé à la valve. La composition est contenue dans ce sac, tandis que l'espace entre le sac et la paroi interne contient un gaz propulseur inerte (air stérile, azote ou CO₂). L'actionnement de la valve comprime le sac et expulse la composition sans contact avec le propulseur, ce qui permet :
(a) un spray multi-position (jusqu'à 360°) et un débit régulier ;
(b) une protection microbiologique de la composition, compatible avec des formulations sans conservateur ;
(c) une stabilité améliorée et une vidange quasi-totale.

Le BOV comprend de préférence :
- une valve anti-retour et un embout nasal ou cutané (droit ou coudé) ;
- un capuchon protecteur et un diffuseur permettant un jet et/ou spray continu (brume) ;
- des matériaux compatibles avec la matrice ionique;
- un conditionnement final en environnement aseptique.

À titre indicatif, la distribution de taille des gouttelettes peut être ajustée pour des usages nasaux ou cutanés, tandis que la pression de service (3-8 bar à 20 °C) et le débit sont choisis pour délivrer une brumisation douce compatible avec l'épithélium.

Selon un mode de réalisation, le conditionnement unidose comprend un récipient unidose (ampoule plastique stérile 2-15 mL ou flacon à valve anti-retour) qui délivre la composition en dose unique ou en multi-doses limitées sans ré-entrée d'air. Cette configuration est adaptée aux situations post-opératoires, aux sujets sensibles aux conservateurs, aux déplacements et à la pédiatrie.

Selon un mode de réalisation, le conditionnement est sous forme de flacon de 200 ml à 2L.

Selon un mode de réalisation, le nébuliseur (à jet, ultrasonique ou membrane vibrante) est configuré pour délivrer la composition sous forme d'aérosol avec une MMAD sélectionnée selon la cible : > 10 µm pour les voies aériennes supérieures et <10 µm pour les voies inférieures. Le kit peut comprendre un embout nasal, une tubulure et, le cas échéant, un masque adulte ou pédiatrique.

### Figures

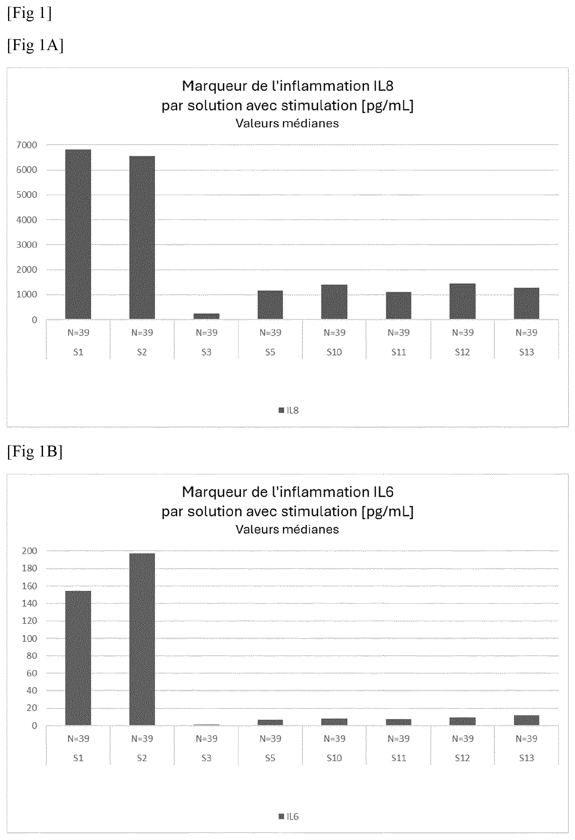

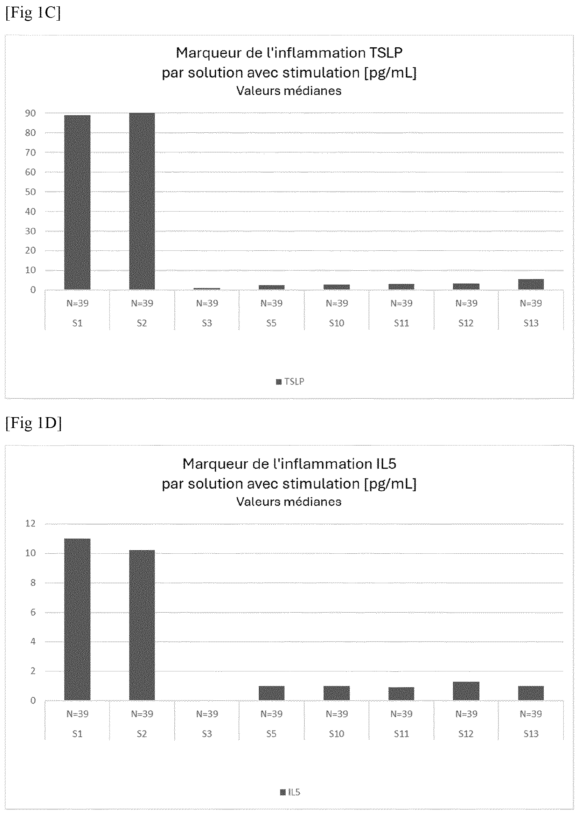

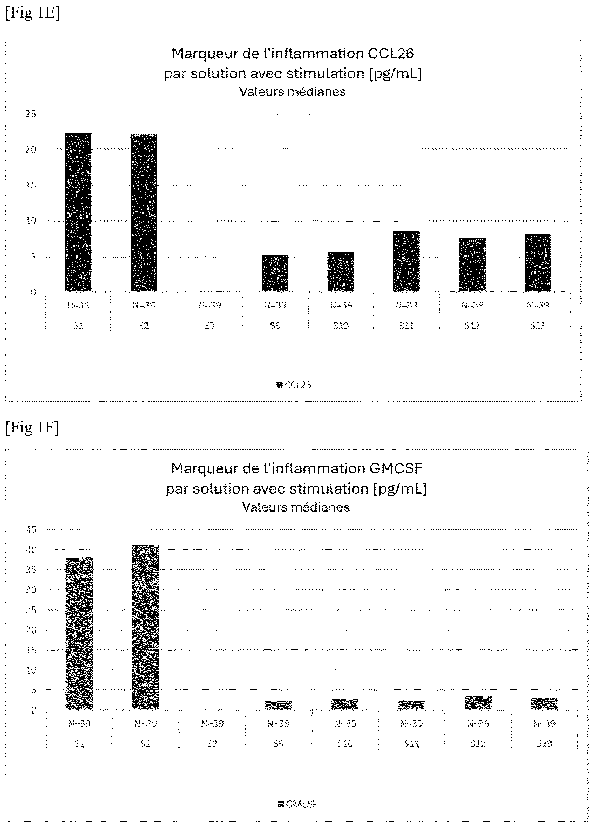

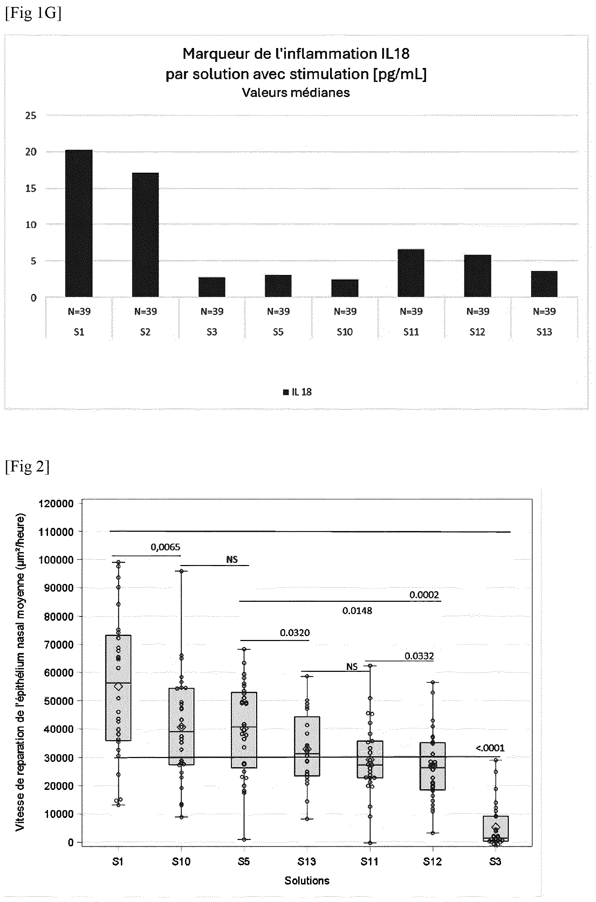

[Fig 1] Graphique représentant les quantités de marqueurs de l'inflammation sécrétées par solution à base d'eau de mer en pg/mL après stimulation, pour les IL8, IL6, TSLP, IL4, IL5, IL13, GM-CSF, IL18, CCL26. Le graphique A représente les secrétions d'IL8 par solution. Le graphique B représente les secrétions d'IL6 par solution (pg/mL). Le graphique représente C représente les secrétions de TSLP par solution. Le graphique D représente les sécrétions d'IL5 par solution . Le graphique E représente les sécrétions de CCL26 par solution (pg/mL). Le graphique F représente les sécrétions de GMCSF par solution (pg/mL). Le graphique G représente les secrétions d'IL18 par solution (pg/mL).
[Fig 2] Graphique présentant la vitesse de réparation de l'épithélium nasal par solution vs. contrôle et NaCl (µm²/heure).

### Définitions

Par « composition ionique » est entendu une composition comprenant des ions, cations et/ou anions, et qui comprend donc notamment du Sodium, du Magnésium, du Calcium, du Potassium, du Chlorure, du Sulfate, du Bicarbonate.

Par « à base d'eau de mer » est entendu que la composition selon l'invention peut être obtenue à partir d'un traitement de l'eau de mer afin d'obtenir une composition ionique particulière.

La « teneur en matière sèche » d'une composition ionique fait référence à la quantité de substances non volatiles qui restent après l'évaporation de l'eau dans une solution contenant des ions. Il s'agit donc de la masse résiduelle des solides, principalement constitués de sels et de minéraux, qui persiste lorsque toute l'eau a été éliminée. Dans le cas d'une solution ionique, comme l'eau de mer par exemple, la matière sèche serait composée des ions dissous (tels que le sodium, le chlorure, le sulfate, etc.) qui, après l'évaporation de l'eau, se retrouvent sous forme de sels solides.

La « résistivité » d'une composition ionique est une propriété physique qui décrit la capacité d'une solution ionique à résister au passage du courant électrique. Elle dépend de la concentration des ions présents dans la solution ainsi que de la nature de ces ions. Plus une solution contient d'ions, plus elle est capable de conduire l'électricité, et donc, sa résistivité est plus faible. À l'inverse, une solution avec moins d'ions aura une résistivité plus élevée.

Par « densité » est entendu la propriété physique décrivant la masse d'un volume donné d'eau. Elle est exprimée dans le cadre de l'invention en grammes par centimètre cube (g/cm³).

L'osmolarité d'une composition ionique est une mesure de la concentration totale des particules osmotiquement actives (principalement des ions) dans une solution. Elle indique combien de moles d'ions ou de molécules sont dissoutes dans un litre de solution et reflète la capacité de la solution à générer une pression osmotique, c'est-à-dire à attirer l'eau à travers une membrane semi-perméable.

Par « isoosmotique » est entendu une composition ionique ayant la même osmolarité que les fluides corporels.

Par « hyperosmotique » est entendu une composition ionique ayant une osmolarité plus élevée que celle des fluides corporels.

Par « agent conservateur » est entendu une substance pour prévenir la croissance de micro-organismes et prolonger la durée de conservation.

La pulvérisation nasale consiste à administrer un médicament ou une solution dans les voies nasales, par exemple à l'aide d'un spray. Cette méthode permet une application locale rapide.

La brumisation nasale est la dispersion d'une fine brume de solution dans les voies nasales à l'aide d'un dispositif. Elle favorise une absorption rapide et uniforme, souvent pour traiter la congestion ou les irritations nasales.

Par « traitement » ou « traiter » est entendu l'atténuation des symptômes associés à un trouble ou un état spécifique et/ou l'élimination desdits symptômes.

Par « prévention » est entendu l'utilisation d'une composition ou d'un médicament pour prévenir l'apparition ou la progression de maladies.

La polypose nasale est une condition où des polypes non cancéreux se forment dans les cavités nasales ou les sinus, entraînant congestion, écoulement nasal, et difficulté à respirer. Ces polypes sont des excroissances gonflées de la muqueuse nasale, souvent associées à des allergies ou à des infections chroniques.

La rhinite allergique est une inflammation des muqueuses nasales causée par une réaction allergique à des allergènes comme le pollen, les acariens ou les poils d'animaux. Elle se manifeste par des symptômes tels que congestion, écoulement nasal, éternuements et démangeaisons.

La dermatite atopique est une maladie de la peau chronique caractérisée par des éruptions cutanées sèches, rouges et démangeaisons. Elle est souvent liée à des allergies et à des facteurs génétiques, et peut affecter n'importe quelle partie du corps.

L'asthme est une maladie respiratoire chronique caractérisée par une inflammation et une constriction des voies respiratoires, provoquant des symptômes comme la toux, la respiration sifflante, l'essoufflement et une oppression thoracique.

Les battements ciliaires sont des mouvements rythmiques effectués par des cils qui tapissent la muqueuse nasale et jouent un rôle crucial dans le système de défense du nez. Leur fonction principale est de déplacer le mucus, qui contient des particules comme la poussière, les allergènes, les microbes et autres impuretés, vers l'arrière de la gorge, où il peut être avalé ou éliminé. Ce mouvement constant aide à maintenir les voies respiratoires propres et à prévenir les infections.

L'irrigation nasale est une pratique médicale consistant à rincer les voies nasales avec une solution saline pour nettoyer les cavités nasales. Cette méthode permet d'éliminer le mucus, les allergènes, les irritants et autres débris accumulés dans le nez.

IL8, IL6, IL18, IL5, IL13, IL 4 sont les acronymes des interleukine 8, 6, 18, 5, 13 et 4.

TSLP signifie Thymic Stromal Lymphopoietin (Lymphopoïétine stromale thymique). C'est une cytokine qui joue un rôle clé dans l'activation des cellules immunitaires, en particulier dans les réponses inflammatoires de type allergique. TSLP est notamment impliquée dans des maladies comme l'asthme, la dermatite atopique.

L'acronyme GM-CSF signifie Granulocyte-Macrophage Colony-Stimulating Factor (Facteur de stimulation des colonies de granulocytes et de macrophages). C'est une cytokine, c'est-à-dire une protéine impliquée les mécanismes de développement des cellules du système immunitaire. Elle joue un rôle clé dans la réponse immunitaire et l'inflammation.

CCL26 signifie C-C Motif Chemokine Ligand 26, aussi appelée éotaxine-3. C'est une chimiokine, une petite protéine impliquée dans le guidage des cellules immunitaires vers les zones d'inflammation ou d'infection. Elle joue un rôle important dans des maladies comme l'asthme ou les allergies.

Par « sujet sain » est entendu un individu humain ne présentant pas de maladies dermatologiques qui seraient traitées par l'administration de la composition selon l'invention, qui permet seulement d'améliorer l'aspect visuel superficiel de la peau et des muqueuses.

Par « application topique », on entend au sens de la présente invention une application sur la peau (dont le cuir chevelu), et les muqueuses.

Par « cosmétiquement acceptable », on entend au sens de la présente invention ce qui est utile dans la préparation d'une composition cosmétique, qui est généralement sûr, non toxique et ni biologiquement ni autrement non souhaitable et qui est acceptable pour une utilisation cosmétique, notamment par application topique au niveau de la peau ou des muqueuses.

Par « non-thérapeutique » est entendu au sens de l'invention, une application cosmétique n'ayant pas vocation à traiter un patient. De fait, la composition cosmétique selon l'invention n'agit pas comme un médicament pour le traitement de pathologies, mais permet d'améliorer l'aspect visuel superficiel de la peau et des muqueuses.

Par « eau purifiée » ou « eau purifiée norme Pharmacopée européenne », on entend une eau répondant à la monographie Ph. Eur. Cette eau peut être obtenue par osmose inverse et/ou échange d'ions, et peut présenter une conductivité ≤ 4,3 µS/cm à 20 °C et un carbone organique total ≤ 0,5 mg/L.Par « électrodialyse » ou « électrodialysée », on entend un procédé membranaire mettant en œuvre un champ électrique et des membranes échangeuses d'ions afin de moduler le profil ionique de l'eau de mer, en ajustant les rapports anions/cations, sans recourir à une évaporation.

Par « radio-décontamination », on entend un traitement par rayonnements ionisants (rayons y ou faisceau d'électrons) appliqué avec une dose absorbée efficace comprise entre 10 et 35 kGy, permettant de maîtriser la biocontamination sans chauffage de la matrice.

Par « bag-on-valve » (ou BOV), on entend un système de conditionnement sous pression comprenant un récipient métallique renfermant un sac barrière souple soudé à une valve. La composition est contenue dans ce sac, tandis que l'espace entre le sac et la paroi interne contient un gaz propulseur inerte (par exemple air stérile, azote ou dioxyde de carbone). Lors de l'actionnement, le gaz comprime le sac et expulse la composition sans contact avec le propulseur, permettant un spray multi-position, une protection microbiologique et une utilisation sans conservateur.

Par « conditionnement unidose », on entend un récipient conçu pour délivrer la composition en dose unique ou en multi-doses limitées sans ré-entrée d'air, par exemple une ampoule plastique stérile ou un flacon équipé d'une valve anti-retour. Ce type de conditionnement est particulièrement adapté aux applications post-opératoires, aux sujets sensibles aux conservateurs et aux usages nomades.

Par « nébuliseur », on entend un dispositif permettant de transformer la composition en aérosol constitué de fines gouttelettes, destiné à une administration par inhalation ou par voie nasale. Le nébuliseur peut être à jet, ultrasonique ou à membrane vibrante, et délivre un aérosol dont la taille des particules (MMAD) est adaptée à la zone cible : typiquement >10 µm pour les voies respiratoires supérieures et <10 µm pour les voies inférieures.

Dans la description et dans les exemples suivants, sauf indication contraire, les pourcentages sont des pourcentages en poids et les plages de valeurs libellées sous la forme « entre ... et ... » incluent les bornes inférieure et supérieure précisées. Les exemples ci-après sont présentés à titre illustratif et non limitatif du domaine de l'invention.

### Exemples

### Exemple 1 : Comparaison in vitro des capacités cicatricielles et anti-inflammatoires de plusieurs solutions dérivées d'eau de mer, riches en minéraux, sur la muqueuse nasale.

### MATERIEL ET METHODES

### Solutions expérimentales :

Huit solutions expérimentales ont été définies avec :
une osmolarité oscillant entre 270 à 360 mOsm/L; par comparaison, les solutions hypertoniques commercialisées sont supérieures à 750 mOsm/L

Des modulations de composition ont été apportées au niveau qualitatif et quantitatif sur les concentrations en chlorures, sodium, calcium, magnésium, soufre et sulfates.

### Trois solutions de contrôle :

S1/A: le milieu de culture des cellules (BEGM) *BEGM^{™} Bronchial Epithelial Cell Growth Medium BulletKit^{™} : Culture system containing BEBMTM Bronchial Epithelial Cell Growth Basal Medium (CC-3171) and BEGMTM Bronchial Epithelial Cell Growth Medium SingleQuotsTM Supplements and Growth Factors (CC-4175) https://bioscience.lonza.com
S2/B : le milieu de culture des cellules et corticoïde, la dexaméthasone.
S3/C : une solution de chlorure de sodium à 0,9% (sérum physiologique). Il s'agit d'un comparateur largement utilisé, pour l'hygiène nasale quotidienne des nourrissons et avec ou sans tampon pour l'irrigation nasale sous des marques telles que Neilmed, Simply Saline, Fess, Arm & Hammer, Simply saline

### Solutions étudiées

Q uatre solutions à base d'eau de mer :
S5/E : Eau de mer diluée obtenue par dilution (procédé Chemineau). Le procédé de dilution et sa mise en œuvre pose la difficulté de la maîtrise de la charge bactérienne. Celle-ci est assurée par une cascade de filtrations pendant tout le process industriel. La particularité de ce process réside dans le fait qu'il ne recoure pas à l'ozonation ni au chauffage de la solution. L'eau de mer utilisée est prélevée en Bretagne (France). La matière première est filtrée et traitée sous UV. Elle subit une série de 5 filtrations successives : 2 filtrations clarifiantes, 2 microfiltrations à 0.2µ (microns) et 1 microfiltration à 0,1µ. Elle est diluée comme suit : 30% d'eau de mer naturelle et 70% d'eau purifiée norme pharmacopée européenne. L'eau de mer est enfin radio décontaminée. En valeur absolue, cette composition est similaire en chlorures de sodium vs. sérum physiologique, sulfates, magnésium, soufre, calcium, potassium, bicarbonates et oligo-éléments (plus de 80) environ à -70% vs. eau de mer.
S10/J: Eau de mer diluée enrichie en sulfate, magnésium, soufre et calcium avec du sulfate de magnésium, lactate de magnésium et sulfate de calcium.
S11/K: Eau de mer électrodialysée, obtenue par électrodialyse (procédé Chemineau)
S12/L: Eau de mer électrodialysée enrichie en sulfate, magnésium, soufre et calcium avec du sulfate de magnésium, lactate de magnésium et sulfate de calcium en partant de S11.

Après analyse intermédiaire, une composition comparative supplémentaire contenant de l'eau de mer électrodialysée (S13) commercialisé sous la marque Physiomer^{®} a été ajouté pour confirmer les résultats inattendus et non conformes vs. littérature

### Composition des solutions

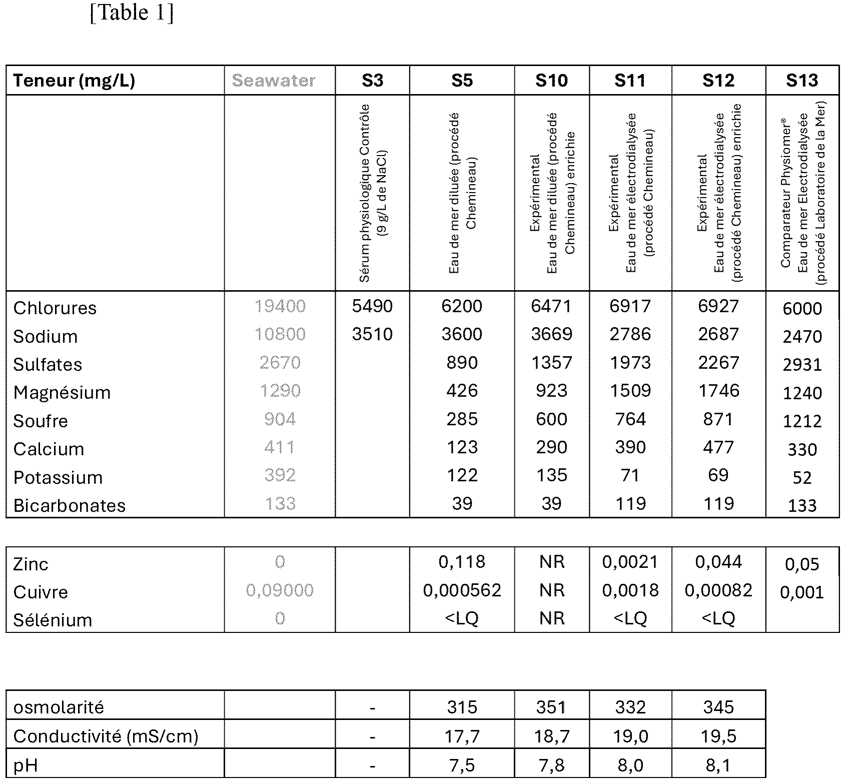

### Design

Etude ex vivo randomisée, aveugle, vs. contrôle et spécialités de référence en comparateur.

### Paramètres étudiés

Les solutions expérimentales produites ont été étudiées sur différents paramètres fonctionnels validés de l'épithélium nasal humain.
la vitesse de réparation des lésions épithéliales
la sécrétion de cytokines pro-inflammatoires.

### Protocole

Les expériences ont été réalisées avec des cellules issues de polypes nasaux de sujets de plus de 50 ans présentant une polypose (rhinosinusite chronique avec polypes) résistante au traitement médical bien conduit de stade III ou IV avec indication chirurgicale (EPOS 2020). Les tissus et cellules de 39 patients différents ont été utilisés. Les polypes nasaux ont été fournis par le service de chirurgie ORL du CHU de Bordeaux (Dr. Ludovic de Gabory). Les prélèvements ont été utilisés et acheminés au laboratoire Inserm UMR-S 1250 selon la règlementation en vigueur.

### 1) Etude de la vitesse de réparation de l'épithélium nasal

Les cellules épithéliales sont isolées des tissus par un traitement enzymatique à la pronase E (Sigma Aldrich). Les cellules sont ensemencées dans des plaques 24 puits (BD Falcon) préalablement coatées avec du collagène de type IV (Sigma Aldrich) dans du milieu de culture CnT17 (CELLnTEC). A 90% de confluence, les cellules sont passées en milieu BEGM (Lonza).

A confluence totale, les cellules sont rincées au PBS (Gibco) puis pré-incubées avec chacune des 12 solutions à tester pendant 4h à 37°C. Une plaie linéaire est alors réalisée dans chaque puits à l'aide d'un cône de pipette de 100µL. Chaque puits est rincé au PBS puis ré-incubé avec la solution de pré-incubation correspondante. Un duplicat technique est réalisé pour chaque solution (2 puits par solution).

Chaque plaie est alors étudiée en vidéomicrocopie (au moins 3 positions différentes le long de la plaie) au grossissement x10, à raison d'une image toutes les 10 minutes, pendant 24 heures. Un plug-in d'ImageJ développé au laboratoire permet de déterminer la vitesse de réparation des lésions en µm2/heure.

A l'issue de l'expérience, les puits sont rincés en PBS et les plaques conservées à -80°C pour une étude ultérieure potentielle de l'expression génique des cellules.

Les cultures de cellules de 39 patients différents ont ainsi été analysées.

### 2) Etude de la sécrétion de cytokines pro-inflammatoires

Les cellules épithéliales isolées des tissus sont ensemencées dans des plaques 48 puits (BD Falcon) préalablement coatées avec du collagène de type IV (Sigma Aldrich) dans du milieu de culture CnT17 (CELLnTEC). A 90% de confluence, les cellules sont passées en milieu BEGM (Lonza).

A confluence totale, les cellules sont rincées au PBS (Gibco) puis pré-incubées pendant 4h avec du milieu de culture BEGM ou avec un mélange de cytokines pro-inflammatoires (IL-1b, TNFa, IFNg) connu pour enflammer les cellules épithéliales respiratoires (22). Les cellules sont alors incubées pendant 4 heures avec chacune des 12 solutions à tester. Un duplicat technique est réalisé pour chaque condition (2 puits pour chaque combinaison d'incubations). Une des solutions (S2) contient un anti-inflammatoire connu (la déxaméthasone) utilisé comme contrôle positif.

A l'issue des différentes incubations, le milieu de culture de chaque puits est collecté. Une analyse multiplexe de son contenu en cytokines est réalisée.

Le reste du milieu de culture est congelé pour une utilisation ultérieure potentielle. Les plaques contenant encore les cellules sont également conservées à - 80°C pour une étude ultérieure potentielle de l'expression génique ou protéique des cellules.

Les cytokines suivantes sont analysées : IL-4, IL-5, IL-6, IL-8, IL-13, IL-25, IL-33, TSLP, GM-CSF, TNFa, éotaxine-3 (CCL26).

Les cultures de cellules de 39 patients différents ont ainsi été analysées.

### Résultats

***Au niveau inflammatoire et des sécrétions d'interleukines et autres substances inflammatoires :***

La Figure 1 présentent les quantités de marqueurs de l'inflammation sécrétées par solution en pg/mL après stimulation, pour les IL8, IL6, TSLP, IL5, CCL26, GM-CSF et IL18
Les TSLP, IL5 et CC26 souvent associés aux réactions allergiques ou à l'asthme ou la dermatite atopique ont été regroupées.
Les interleukines IL 25, 33, 13 et 4 n'ont pas été représentées du fait de l'absence ou des très faibles quantités sécrétées.

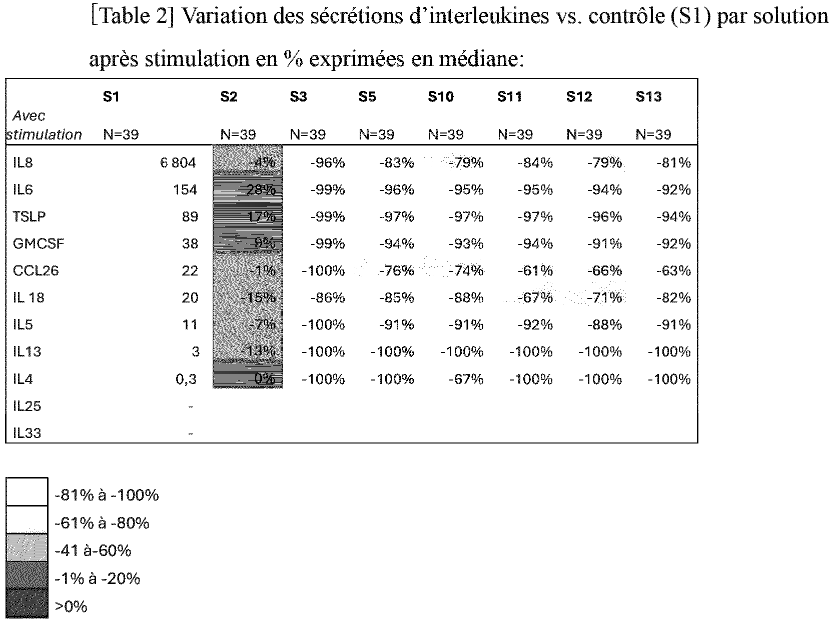

Une large palette d'interleukines (IL) et de molécules impliqués en tant que médiateurs de la réponse inflammatoire et immunitaire ont été étudiées, et en particulier les TSLP, CCL26, IL-4, IL-5, IL-13 qui sont spécifiquement impliqués dans la réponse immunitaire de type Th2, et la réponse allergique et asthmatique.

Des interleukines moins spécifiques de l'inflammation et l'immunité comme l'IL8 ont également été étudiées.

Le milieu de culture des cellules (BEGM) Bronchial Epithelial Cell Growth Medium (S1) ne montre pas d'activité anti-inflammatoire ce qui n'est pas surprenant.

De manière inattendue, le contrôle (S2) contenant un corticoïde puissant, la dexaméthasone, ne montre aucune réduction de la production d'interleukines ; notre hypothèse est que les effets initiaux de la corticothérapie nasale sont souvent modestes. L'effet complet et optimal peut prendre 1 à 2 semaines d'utilisation régulière. Pour être efficace, la corticothérapie nasale nécessite une bonne adhérence, une utilisation non sporadique.

Toutes les solutions expérimentales testées montrent une baisse spectaculaire des sécrétions d'interleukines vs. contrôle (S1) et (S2) contenant de la dexaméthasone.

Une particularité de ces résultats est qu'ils sont observés rapidement entre 12 et 24h. On perçoit l'intérêt d'une composition contenant des corticoïdes au sein d'une des solutions expérimentales riches en électrolytes pour obtenir un effet immédiat avant que les corticoïdes n'agissent et permettent d'améliorer l'adhérence de ces traitements et leur efficacité.

Les résultats quasi imperceptibles sur sérum physiologique (S3) peuvent refléter l'apoptose des cellules épithéliales : les cellules épithéliales respiratoires connaissent un phénomène de paralysie ou mort cellulaire. Cette solution est à écarter en usage respiratoire.

Les minéraux et oligo-éléments jouent un rôle crucial dans le système antioxydant de l'organisme, aidant à neutraliser les radicaux libres et à prévenir les dommages cellulaires en particulier le magnésium, le soufre

Une particularité de ces résultats est la nature anti-inflammatoire globale non spécifique. On observe un effet sur 7 des 11 marqueurs de l'inflammation testés: IL8, IL6, TSLP, GM-CSF, eotaxine-3, IL18, IL5.

Toutes les interleukines montrent des affaissements voire suppressions spectaculaires vs. contrôle (milieu), quelle que soit la solution, et de manière très intéressante les TSLP, CCL26, IL-5. Ces interleukines sont souvent associée aux réactions allergiques et à l'asthme. Les IL-5, -4 et -13 ne sont pas produites par les cellules épithéliales mais par les cellules immunitaires majoritairement ce qui peut expliquer qu'elles se soient peu ou pas exprimées.

L'IL 8 est l'interleukine sécrétée le plus largement quantitativement par les cellules nasales en pg/mL ; on voit des réductions spectaculaires vs. S1 et S2 dexaméthazone. L'IL-8 joue un rôle important dans les réponses immunitaires aiguës aux infections bactériennes et virales. Une production excessive d'IL-8 peut contribuer à l'inflammation chronique et aux dommages tissulaires dans des maladies comme la polyarthrite rhumatoïde, la maladie de Crohn, et la maladie pulmonaire obstructive chronique (MPOC).

Les solutions montrent des réductions spectaculaires sur les IL6, TSLP, GM-CSF, eotaxine-3, IL18, IL5 (figure 1)

L'IL-6 est impliquée dans la régulation de l'inflammation, de l'immunité, et du métabolisme. On la retrouve dans de nombreuses maladies inflammatoires chroniques, auto-immunes, et certains cancers. Des thérapies ciblant l'IL-6 ou son récepteur (IL-6R) ont été développées, par exemple, le tocilizumab, un anticorps monoclonal qui inhibe l'IL-6R, est utilisé pour traiter la polyarthrite rhumatoïde et d'autres maladies inflammatoires.

Le TSLP est fortement exprimé dans les voies respiratoires hautes (rhinite allergique où il participe à l'inflammation des voies nasales) et dans les voies respiratoires basses chez des patients asthmatiques. Il joue un rôle clé dans l'initiation et la maintenance de l'inflammation allergique dans l'asthme. Il est également impliqué dans la dermatite atopique où il est surexprimé dans la peau, contribuant à l'inflammation chronique et à la sensibilité accrue aux allergènes. Son expression peut être augmentée en réponse à des infections virales ou des irritants environnementaux, amplifiant ainsi l'inflammation. C'est une cible pour le développement de nouveaux traitements pour les maladies allergiques et inflammatoires. Par exemple, des anticorps monoclonaux ciblant le TSLP, comme le tezepelumab, pour traiter des conditions comme l'asthme sévère ou le mepolizumab (Astra Zeneca) dans la polypose.

Le GM-CSF est impliqué dans la pathologie de la PR, Polyarthrite rhumatoïde, où il contribue à l'inflammation et à la destruction des articulations et dans certaines maladies pulmonaires inflammatoires, comme la pneumonie interstitielle et la protéinose alvéolaire pulmonaire, où il est essentiel pour le maintien des macrophages alvéolaires, qui nettoient les poumons des débris cellulaires. Bien qu'il soit bénéfique pour combattre les infections et soutenir le système immunitaire, il peut également contribuer à des conditions inflammatoires chroniques et auto-immunes.

La CCL26 (éotaxine-3) est une chimiokine principalement impliquée dans le recrutement des éosinophiles vers les sites d'inflammation en particulier dans les maladies allergiques et inflammatoires comme l'asthme, la rhinite allergique et la dermatite atopique.

L'IL-5 est une cytokine impliquée dans la régulation des éosinophiles et dans les réponses immunitaires contre les parasites et dans les maladies allergiques, telles que l'asthme et la rhinite allergique. Plusieurs traitements ciblant l'IL-5 ont été développés. Par exemple, le mépolizumab et le reslizumab sont des anticorps monoclonaux qui neutralisent l'IL-5, réduisant ainsi le nombre d'éosinophiles et atténuant les symptômes chez les patients souffrant d'asthme éosino-philique sévère..

Les résultats présentés permettent d'envisager une utilisation de ces solutions seules ou avec d'autres ingrédients en dispositif médical pour tout tableau impliquant une forme d'inflammation ou d'allergie

Les résultats présentés permettent d'envisager une utilisation de ces solutions seules ou avec d'autres ingrédients en cosmétique pour tout tableau impliquant des peaux à tendance allergique, ou à tendance atopique et les signes associés tels que irritation, ou démangeaison

### Vitesse de réparation de l'épithélium nasal (cicatrisation) :

La figure 2 présente la vitesse de réparation de l'épithélium nasal par solution vs. contrôle (S1) et NaCl (S3)(µm²/heure)

La solution (S3), qui est du sérum physiologique NaCL 0.9%, montre des résultats très significativement inférieurs à toutes les solutions. Le phénomène d'apothose est confirmé par vidéomicroscopie.

Des différences inattendues sont visibles sur cette figure au niveau de la cicatrisation.

Le milieu de culture BEGM (S1) donne des résultats supérieurs à toutes les autres solutions.

Les solutions (S5), (S10) démontrent ensuite une efficacité supérieures vs. le produit comparateur du marché (S13) pouvant indiquer que le mode de fabrication par dilution de l'eau est plus intéressant et plus efficace pour favoriser la cicatrisation

Aucune différence entre (S10), et (S5) n'a été constaté.

La solution (S12) démontre une efficacité inférieure à (S11), (S13), (S5) et (S10). On notera la valeur particulièrement significative entre (S5) et (S12).

### Résumé et Discussion

Les différentes expérimentations réalisées ci-dessus ont permis d'identifier l'efficacité des compositions étudiées pour des dispositifs médicaux dans des indications topiques anti-inflammatoires, anti-allergiques, et dans la cicatrisation.

Les différentes expérimentations réalisées ci-dessus ont permis d'identifier l'efficacité des compositions étudiées pour des cosmétiques topiques visant des effets apaisants et réparateurs notamment sur des peaux irritées, ou à tendance atopique, ou en suivi de soins post-chirurgicaux, voire en soins anti-âge .

**[Table 3] Performances des solutions suivant les paramètres observés**

| | **IL8, IL6, TSLP, IL5, GM-CSF, IL18, eotaxine-3** | **Vitesse de cicatrisation (µm²/heure)** |
|---|---|---|
| S1 | | X |
| S3 | | |
| S5 | X | X |
| S10 | X | X |
| S11 | X | |
| S12 | X | |
| S13 | X | |

L'effet sur les IL8, IL6, TSLP, IL5, GM-CSF, IL18, eotaxine-3, est comparable entre (S5), (S10), (S11), (S 12) et (S13) ; on ne voit se dégager de différence significative.

L'effet favorable sur la vitesse de cicatrisation est comparable entre les deux solutions diluées (S5) et (S10) bien que seule (S5) montre une supériorité significative vs. (S12) le comparateur obtenu par électrodialyse.

La composition (S5) et (S10) démontrent une efficacité dans des indications anti-inflammatoires et dans la cicatrisation.

Il se dégage en effet de cette étude une double activité: cicatricielle et anti-inflammatoire au-delà des marqueurs traditionnels IL 8 et IL6, pour la compositions (S5) supérieure au comparateur.

Cette composition a une action anti-inflammatoire sur 7 des marqueurs testés de l'inflammation : IL8, IL6, TSLP, IL5, GM-CSF, IL18, eotaxine-3.

On observe cette activité anti-inflammatoire sur des marqueurs impliqués dans l'allergie et les pathologies respiratoires chroniques telles que la polypose ou l'asthme : TSLP, IL 5.

Ces effets sont significatifs en comparaison à un corticoïde la dexaméthasone et à fortiori sur les corticoïdes partageant le même mode d'action

Ces effets sont rapides et ont été constatés en 12 à 24 heures.

Des baisses spectaculaires des marqueurs de l'inflammation sont constatées.

Les propriétés de l'irrigation dans la prévention du rhume ouvrent la voie à des applications prophylactiques sur l'ensemble des pathologies respiratoires en particulier dans le contexte des allergies, de l'asthme, de la pollution atmosphérique et de la chronicisation des maladies.

Une utilisation en association ou en appoint est envisageable, avec des corticoïdes ou anticorps monoclonaux, notamment ceux utilisés dans la polypose qui agissent sur la réduction de l'IL 5.

En outre, ces résultats suggèrent une utilisation dans les maladies immunitaires impactant la santé de poumons comme la polyarthrite rhumatoïde, en association ou appoint des anti-fibrosants dans la fibrose pulmonaire, dans les thérapies ou la prévention des pathologies respiratoires basses telles que les bronchites aiguës, les pneumopathies aiguës, l'asthme, les pneumopathies chroniques, BPCO, les surinfections de bronchites chroniques obstructives, les maladies pulmonaires professionnelles..., ou le RGO

Ces résultats suggèrent également des applications en nébulisation dans la BPCO, les bronchites, l'asthme.

Les composition (S5) et (S10) constituées d'eau de mer diluée à 70% sont supérieures à toutes les solutions. Ces compositions présentent un effet cicatriciel significativement plus important par rapport à celles obtenues par électrodialyse. Le procédé de fabrication des sprays d'eau de mer impacte ainsi la vitesse de cicatrisation.

### Références

Mishra V, Banga J, Silveyra P. Oxidative stress and cellular pathways of asthma and inflammation: Therapeutic strategies and pharmacological targets. Pharmacol Ther. 2018 Jan;181:169-182.
Topal O, Kulaksizoglu S, Erbek SS. Oxidative stress and nasal polyposis: does it affect the severity of the disease? Am J Rhinol Allergy. 2014 Jan-Feb;28.
Hong Z, Guo Z, Zhang R, Xu J, Dong W, Zhuang G, Deng C. Airborne Fine Particulate Matter Induces Oxidative Stress and Inflammation in Human Nasal Epithelial Cells. Tohoku J Exp Med. 2016 Jun;239(2):117-25.
Bayram H, Devalia JL, Sapsford RJ, et al. The effect of diesel exhaust particles on cell function and release of inflammatory mediators from human bronchial epithelial cells in vitro. Am J Respir Cell Mol Biol. 1998;18:441-448.
Calderon-Garciduenas L, Valencia-Salazar G, Rodriguez-Alcaraz A, et al. Ultrastructural nasal pathology in children chronically and sequentially exposed to air pollutants. Am J Respir Cell Mol Biol. 2001;24:132-138.
Gosepath J, Grebneva N, Mossikhin S, Mann WJ. Topical antibiotic, antifungal, and antiseptic solutions decrease ciliary activity in nasal respiratory cells. Am J Rhinol. 2002;16:25-31.
Janson H, Carl'en B, Cervin A, et al. Effects on the ciliated epithelium of protein D-producing and -nonproducing non-typeable Haemophilus influenza in nasopharyngeal tissue cultures. J Infect Dis. 1999;180:737-746.
Read RC, Roberts P, Munro N, et al. Effect of Pseudomonas aeruginosa rhamnolipids on mucociliary transport and ciliary beating. J Appl Physiol. 1992;72:2271-2277.
Read RC, Rutman AA, Jeffery PK, et al. Interaction of capsulate Haemophilus influenzae with human airway mucosa in vitro. Infect Immun. 1992;60:3244-3252.
Steinfort C,Wilson R, Mitchell T, et al. Effect of Streptococcus pneumoniae on human respiratory epithelium in vitro. Infect Immun. 1989;57:2006-2013.
Holmström M, Lund V, Scadding G. Nasal ciliary beat frequency after nasal allergen challenge. Am J Rhinol. 1992;6:101-105.
MauriziM, Paludetti G, Todisco T, et al. Ciliary ultrastructure and nasal mucociliary clearance in chronic and allergic rhinitis. Rhinology. 1984;22:233-240.
Mezey RJ, Cohn MA, Fernandez RJ, et al. Mucociliary transport in allergic patients with antigen-induced bronchospasm. Am Rev Respir Dis. 1978;118:677-684.
Ohashi Y, Nakai Y, Kihara S, et al. Ciliary activity in patients with nasal allergies. Arch Otorhinolaryngol. 1985;242:141-147.
Tomooka LT, Murphy C, Davidson TM. Clinical study and literature review of nasal irrigation. Laryngoscope. 2000;110:1189-1193.
Slapak I, Skoupá J, Strnad P, Hornik P. Efficacy of isotonic nasal wash (seawater) in the treatment and prevention of rhinitis in children. Arch Otolaryngol Head Neck Surg. 2008;134(1):67-74.
Tano L, Tano K. A daily nasal spray with saline prevents symptoms of rhinitis. Acta Otolaryngol. 2004 Nov;124(9):1059-62.
King D, Mitchell B, Williams CP, Spurling GK. Saline nasal irrigation for acute upper respiratory tract infections. Cochrane Database Syst Rev. 2015 Apr 20;(4):CD006821
Rudmik L, Hoy M, Schlosser RJ, Harvey RJ, Welch KC, Lund V, Smith TL. Topical therapies in the management of chronic rhinosinusitis: an evidence-based review with recommendations. Int Forum Allergy Rhinol. 2013 Apr;3(4):281-98
Gallant JN, Basem JI, Turner JH, et al. Nasal saline irrigation in pediatric rhinosinusitis: A systematic review. Int J Pediatr Otorhinolaryngol. 2018 May;108:155-162.
Hermelingmeier KE, Weber RK, Hellmich M, et al. Nasal irrigation as an adjunctive treatment in allergic rhinitis: a systematic review and meta-analysis. Am J Rhinol Allergy. 2012;26:e119-e125.
Roberts G, Xatzipsalti M, Borrego LM, et al. Paediatric rhinitis: position paper of the European Academy of Allergy and Clinical Immunology. Allergy. 2013;68:1102-1116.
Angier E, Willington J, et al. Management of allergic and non-allergic rhinitis: A primary care summary of the BSACI (British Society of Allergy and Clinical Immunology) guideline. 2010.
Chia-Ling Li, Hsiao-Chuan Lin, Chien-Yu Lin, and Teh-Fu Hsu. Effectiveness of Hypertonic Saline Nasal Irrigation for Alleviating Allergic Rhinitis in Children: A Systematic Review and Meta-Analysis. J Clin Med. 2019 Jan; 8(1): 64.
Head K, Snidvongs K, Glew S, et al. Saline irrigation for allergic rhinitis. Cochrane Database of Systematic Reviews. June 2018.
Scadding G.K, Kariyawasam H.H, et al. BSACI guideline for the diagnosis and management of allergic and non-allergic rhinitis. Clin Exp Allergy. 2017;47:856-889.
Wang YH, KuMS, Sun HL, Lue KH. Efficacy of nasal irrigation in the treatment of acute sinusitis in atopic children. J Microbiol Immunol Infect. 2014;47:63-69.
Wang YH, Yang CP, Ku MS, et al. Efficacy of nasal irrigation in the treatment of acute sinusitis in children. Int J Pediatr Otorhinolaryngol. 2009;73:1696-1701.
Rosenfeld RM et al. Clinical practice guideline (update): Adult Sinusitis Executive Summary. Otolaryngol Head Neck Surg. 2015 Apr;152(4):598-609
Fokkens WJ, Lund VJ, Mullol J, et al. European Position Paper on Rhinosinusitis and Nasal Polyps 2012. Rhinol Suppl. 2012;(23):3 p preceding table of contents, 1-298.
Seppey 1996
Holmstrom 1997
Slapak I, Skoupá J, Stmad P, Hornik P. Efficacy of isotonic nasal wash (sea-water) in the treatment and prevention of rhinitis in children. Arch Otolaryngol Head Neck Surg. 2008;134(1):67-74.
Culig 2010
Hahn 2013
Salib 2013
Tugrul 2015
Atar 2022
Chen 2014

## Revendications

1. Composition à base d'eau de mer ayant :
- un pH de 7 à 8 ;
- une conductivité de 15 à 19,5 mS/cm ;
- une osmolarité de 280 à 370 mOsm/kg, de préférence de 290 à 360 mOsm/kg ;
et comprenant la teneur suivante en les principaux constituants :
- 2100 à 3900 mg/L de sodium (Na)
- 6000 à 7000 mg/L de chlorure (Cl)
- 20 à 120 mg/L de bicarbonate (HCO₃)
- 400 à 1900 mg/L de magnésium (Mg) ;
- 100 à 650 mg/L de calcium (Ca) ;
- 45 à 140 mg/L de potassium (K) ;
- 700 à 2600 mg/L de sulfate (SO₄).

2. Composition selon la revendication précédente, comprend en outre :
- 200 à 3000 mg/L de soufre (S).

3. Composition selon l'une quelconque des revendications précédentes, comprend en outre au moins un parmi : du zinc (Zn), du cuivre (Cu), du sélénium (Se), du manganèse (Mn), du fer (Fe).

4. Composition selon l'une quelconque des revendications précédentes, qui est dépourvue de tout agent conservateur.

5. Composition selon l'une quelconque des revendications précédentes, dont les principaux constituants sont :
- 3600 mg/L de sodium (Na) ;
- 6200 mg/L de chlorure (Cl) ;
- 39 mg/L de bicarbonate (HCO₃)
- 426 mg/L de magnésium (Mg) ;
- 123 mg/L de calcium (Ca) ;
- 122 mg/L de potassium (K) ;
- 890 mg/L de sulfate (SO₄) ;
- 285 mg/L de soufre (S).

6. Composition selon l'une quelconque des revendications 1 à 4, comprenant :
- 3669 mg/L de sodium (Na) ;
- 6471 mg/L de chlorure (Cl) ;
- 39 mg/L de bicarbonate (HCO₃)
- 923 mg/L de magnésium (Mg) ;
- 290 mg/L de calcium (Ca) ;
- 135 mg/L de potassium (K) ;
- 1357 mg/L de sulfate (SO₄) ;
- 600 mg/L de soufre (S).

7. Composition selon l'une quelconque des revendications 1 à 6, pour utilisation dans le traitement ou la prévention des affections respiratoires en cas d'état inflammatoire, allergique ou dans la cicatrisation, .

8. Composition selon l'une quelconque des revendications 1 à 6, pour utilisation dans le traitement ou la prévention de la polypose nasale, la rhinite allergique, ou l'asthme.

9. Composition selon l'une quelconque des revendications 1 à 6, pour utilisation dans le traitement ou la prévention des pathologies dermatologiques nécessitant un effet anti-inflammatoire et/ou de cicatrisation.

10. Composition selon l'une quelconque des revendications 1 à 6, pour utilisation, sous une forme adaptée à une administration par voie topique, sur la peau ou les muqueuses.

11. Composition selon l'une quelconque des revendications 1 à 6, pour utilisation, sous une forme adaptée à une administration par voie topique pour rincer, nettoyer et/ou assainir la peau et/ou les muqueuses.

12. Composition selon l'une quelconque des revendications 1 à 6, pour utilisation, sous une forme adaptée à une administration par voie topique, pour favoriser la réparation de la peau ou des muqueuses, ou favoriser la fonction barrière de la peau ou des muqueuses, ou favoriser la réduction du stress oxydatif de la peau et/ou des muqueuses.

13. Composition selon l'une quelconque des revendications 1 à 6, pour utilisation, sous une forme adaptée à une administration par voie topique, pour soulager les rougeurs, gonflements, irritations, démangeaisons, suintements, grattages, ou sensations de brûlures

14. Composition pour utilisation selon l'une quelconque des revendications 1 à 6, pour favoriser la cicatrisation de tissus épithéliaux, de préférence de la muqueuse nasale, où ladite composition est diluée dans de l'eau déminéralisée à un ratio entre 3% et 35%, de préférence 5 % ou 15 %, ou 30% (m/m).

15. Dispositif médical destiné à l'administration d'une composition ionique à base d'eau de mer selon l'une quelconque des revendications 1 à 6.
